# EUROPEAN PATENT SPECIFICATION

(11) **EP 2 054 029 B1**
(45) Date of publication and mention of the grant of the patent: **23.06.2010**
(21) Application number: 07788135.7
(22) Date of filing: 01.08.2007
(51) Int. Cl.: A61K 9/00

(54) **SUBCUTANEOUS IMPLANTS RELEASING AN ACTIVE PRINCIPLE OVER AN EXTENDED PERIOD OF TIME**
SUBKUTANE IMPLANTATE, DIE ÜBER EINEN LÄNGEREN ZEITRAUM EINEN WIRKSTOFF FREISETZEN
IMPLANTS SOUS-CUTANÉS LIBÉRANT UN PRINCIPE ACTIF PENDANT UNE DURÉE ÉTENDUE

(30) Priority: 02.08.2006 IT MI20061538
(43) Date of publication of application: 06.05.2009
(73) Proprietor: Mediolanum Pharmaceuticals Limited, Dublin 4 (IE)
(72) Inventor: MAURIAC, Patrice, 75012 Paris (FR); MARION, Pierre, 93360 Neuilly Plaisance (FR)
(74) Representative: Cattaneo, Elisabetta
(86) International application number: PCT/EP2007/057967
(87) International publication number: WO 2008/015236

(56) References cited:
- WO-A-00/33809
- WO-A-2005/110368
- US-A1- 2003 211 157

## Description

### Field of the invention

The present invention relates to subcutaneous implants releasing an active principle over an extended period of time, containing an active ingredient dispersed in a polymeric matrix of PLGA, obtained by grinding an extruded product of a blend of at least two PLGA having different lactic acid/glycolic acid molar ratios and different weight average molecular weights or a PLGA and PLA having different weight average molecular weight and the relative process for preparing said implants.

### State of the art

Many therapeutic agents are rapidly metabolized and eliminated by the human or mammalian organism, therefore requiring frequent administration of the drug with the aim of maintaining an adequate therapeutic concentration.

The advantage of using implants containing controlled release drugs is well known in the state of the art.

Among the numerous subcutaneous implants known in the art, those described in WO00/33809 represent a net improvement with reference to previous subcutaneous implants containing, as the active principle, a polypeptide dispersed in a matrix of polylactic-glycolic acid in that they are able to release the aforesaid active principle in 6 months. The subcutaneous implants described in said previous patent application differ also in that they present an essentially triphasic and not biphasic release profile, namely a release by pure diffusion, controlled diffusion following swelling and release by polymer degradation

This progression therefore allows for an extension of release times. In fact when these implants are introduced into an aqueous medium, the water diffuses through the polymeric matrix reaching the peptide particles closest to the surface and subsequently the inner zones.

The implant remains substantially unmodified for about 6 weeks and in this period releases approximately 30% of the peptide.

The duration of this stage of pure diffusion is essentially determined by the level of heterogeneity of the peptide dimensions and the rate is essentially determined by the particle content in the PLGA matrix.

As the active principle presents heterogenous particles of dimensions, a sufficient quantity of peptide remains after the first stage of dissolution and can be released in the successive stages mentioned, that is release by diffusion and swelling, and release by disintegration of the polymer.

All the subcutaneous implants including those previously mentioned suffer from a drawback essentially caused by the fact that once the same are administered in the human body, mainly in the first days they release daily a considerably high overall amount of active principle (in some cases decidedly higher than maximum permitted daily dosages).

This is essentially caused by an immediate dissolution of the active principle, this phenomenon, which does not deplete in subsequent days but some times increases in a scalar progression, is known as initial "burst". In such cases, therefore, it can be verified that the quantity of drug released from such systems, although if compared to the quantity of total active principle contained in the subcutaneous implants administered is low, can in some cases be considered dangerous if with such an initial burst the maximum permitted daily dosage for such a type of drug is approached or exceeded.

Subcutaneous implants having limited initial release of the active principle and consequently a linearly varying release thereof consisting of:
a core (i) comprising an active principle dispersed in a polymeric matrix of polylactic-glycolic acid (PLGA) copolymer,
a coating in film form (ii), essentially consisting of a lactic-glycolic acid copolymer, and the relative processes for preparing said implants are described in WO 2005/000278 A1.

### Summary of the invention

The Applicant has now unexpectedly found subcutaneous PLGA based implants formulations which overcome the above drawback, namely lack of linearity of the release profile and important initial release rate or burst release, without the necessity to coat said implants as imposed in the aforementioned WO2005/000278 A1. The present invention therefore relates to subcutaneous implants obtained by extrusion containing an active ingredient dispersed in a PLGA matrix, wherein said matrix is obtained by grinding an extruded product consisting of a blend of:
- at least two PLGA having different lactic acid/glycolic acid molar ratios and different weight average molecular weights, or
- a PLGA and PLA having different weight average molecular weight.

Such implants result to release their active ingredient on a fairly linear way or, more generally speaking, in a way that could fit in with a specific set of criteria for a particular formulation purpose (e.g. increasing the overall release duration by limiting the initial burst release).

### Description of the figures

Figure 1 shows, in ordinates, the active ingredient overall release profile expressed in mg versus, in abscissa, the time expressed in days after immersion in the aqueous medium of the subcutaneous implants prepared as described in Example 1.
Figure 2 shows, in ordinates, the active ingredient overall release expressed in % of total, versus, in abscissa, the time expressed in days after immersion in the aqueous medium of the sole subcutaneous implant 1#3 prepared as described in Example 1.
Figure 3 reports, in ordinates, the active ingredient overall release profile expressed in mg, versus, in abscissa, the time expressed in days after immersion in the aqueous medium of the subcutaneous implants prepared as described in Example 2.
Figure 4 reports, in ordinates, the active ingredient overall release expressed in mg versus, in abscissa, the time expressed in days after immersion in the aqueous medium of the subcutaneous implants prepared as described in Example 3.
Figure 5 reports, in ordinates, the active ingredient overall release expressed in mg versus, in abscissa, the time expressed in days after immersion in the aqueous medium of the subcutaneous implants prepared as described in Example 4,.
Figure 6 reports, in ordinates, the active ingredient overall release expressed in mg versus, in abscissa, the time expressed in days after immersion in the aqueous medium of the subcutaneous implants prepared as described in Example 5.

### Detailed description of the invention

The subcutaneous implants of the present invention contain active principles chosen from the group consisting of: a peptide, an active principle able to increase bone density, an analgesic-narcotic active principle, a steroid hormone, for hormone treatments during menopause and for contraception.

Preferably said peptide is chosen from: avorelin, triptorelin, goserelin and leuprorelin.

The active ingredient able to increase bone density are chosen from: pharmaceutically acceptable bisphosphonic acids and their salts, vitamin D and sex hormones.

Of these bisphosphonic acids and their pharmaceutically acceptable related salts, we mention for example the compounds of general formula (I): 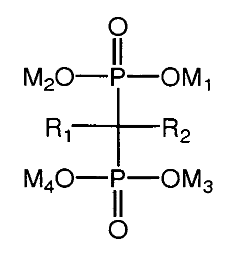 in which M₁, M₂, M₃ and M₄ are monovalent cations and/or H, where said monovalent cations are chosen from alkaline metals, or cations of aliphatic or cycloaliphatic amines, and even more preferably said cations are Na⁺, we would cite for example those in which R₁ and R₂ have the meanings given in the following table 1:

**Table 1**

| Bisphosphonate | R₁ | R₂ |
|---|---|---|
| Etidronate | OH | CH₃ |
| Chlodronate | Cl | Cl |
| Pamidronate | OH | CH₂CH₂NH₂ |
| Alendronate | OH | CH₂ CH₂ CH₂NH₂ |
| Risedronate | OH | CH₂-3-pyridine |
| Tiludronate | H | CH₂-S-phenyl-4Cl |
| Ibandronate | OH | CH₂CH₂N(CH₃)pentyl |
| Zoledronate | OH | CH₂CH₂-1-imidazole |
| Minodronate | OH | CH₂CH₂-2-imidazopyridinyl |
| Incadronate | OH | N-(cycloheptyl) |
| Olpadronate | OH | CH₂CH₂N(CH₃)₂ |
| Neridronate | OH | CH₂CH₂CH₂CH₂CH₂NH₂ |
| EB1053 | OH | CH₂-1-pyrrolidinyl |

Particularly preferred are disodium etidronate, disodium alendronate and disodium pamidronate.

Preferably the sex hormones are selected from the group of estrogens and progestins and of the latter, androgenic progestins are preferably used.

Estrogens are of steroid type preferably chosen from the class consisting of estradiol, estradiol valerate, estradiol cypionate, estrone, estrone sulphate or estrogens of non-steroidal type for example diethylstilbestrol, p-p'-DDT, bisphenyl-A.

Among male progestins those preferred are chosen from the class consisting of norethindrone, norethinodrel, norgestrel, desogestrel, norgestimate.

As "drugs with narcotic analgesic activity" preferred are morphine and morphinans, i.e. compounds having a chemical structure and activity similar to that of morphine i.e. µ receptor agonists, but also compounds with morphinic-type activity, in other words also µ receptor agonists but with a different chemical structure such as those belonging to the phenylpiperidine class. (Goodman & Gilman's "The pharmacological basis of therapeutics "Ninth Edition Chapter 23 pages 521-555). As phenylpiperidine µ receptor agonists we cite as preferred at least one active principle chosen from the class consisting of meperidine, fentanyl and relative pharmaceutically acceptable salts, fentanyl congeners, for example sufentanyl, alfentanyl, lofentanyl, carfentanyl, remifentanyl and their pharmaceutically acceptable salts.

The active principle present in the subcutaneous implants of the invention can present heterogeneous dimensions or can have a more homogeneous particle size distribution.

Preferably, when the subcutaneous implants according to the present invention contain a peptide as the active ingredient, they show a heterogeneous size distribution more preferably ranging from 1 to 63µm or from 1 to 100 µm. Specifically, when the subcutaneous implants of the invention contain the peptides having the aforesaid heterogenous particles size dimensions, the resulting extruded blended PLGA contained in the subcutaneous implants according to the present invention has preferably a lactic acid / glycolic acid molar ratio ranging from 50/50 and 90:10 and the weight average molecular weight ranges from 50000 to 150000.

The subcutaneous implants according to the present invention are prepared with a process comprising the following steps
a) Mixing at least two PLGA having different weight average molecular weight and different lactic acid/ glycolic acid molar ratio,
b) extruding the powder mix coming from step (a) and then grinding the extruded PLGA mixture, thereby obtaining granules of the blended extruded PLGA
c) dry mixing the active agent in the form of particles with the granules of blended extruded PLGA obtained in step (b) or (c') wet granulating the particles of said active ingredient and the granules of extruded blended PLGA coming from step (b) in the pretence of a suitable solvent, such as water or lower alcohol,
d) drying the granulated product coming from the wet granulation of step (c') thereby obtaining a residue containing a maximum liquid content of between 0.1 and 3%,
e) extruding the dry mixture obtained in step (c) or the dried granulated product from step (d).

We report herewith for illustrative but not limiting purposes the following examples of the subcutaneous implants according to the present invention.

### EXAMPLE 1- preparation of subcutaneous implants containing Goserelin (formulations No. 1#1, 1#2 and 1#3)

Subcutaneous implants containing 23.5% w/w Goserelin (having particle size distribution ranging from 1 to 63 µm) and PLGA having compositions, UG molar ratios and molecular weights as defined in the table below are prepared as described in WO00/33809

| | PLGA mix composition | Resulting "blended" PLGA | |
|---|---|---|---|
| | | L/G molar ratio | Molecular weight |
| 1#1 | 1 single PLGA | 59/41 UG | 60 kg/mol |
| 1 #2 | 2 PLGAs: - 25% m/m of a 72/28 UG - 118 Kg/mol PLGA - 75% m/m of a 54/46 UG - 51 Kg/mol PLGA | | |
| 1 #3 | 3 PLGAs: - 37.5% m/m of a 72/28 L/G - 118 Kg/mol PLGA - 37.5% m/m of a 54/46 UG - 51 Kg/mol PLGA - 25% m/m of 51/49 L/G - 17 Kg/mol | | |

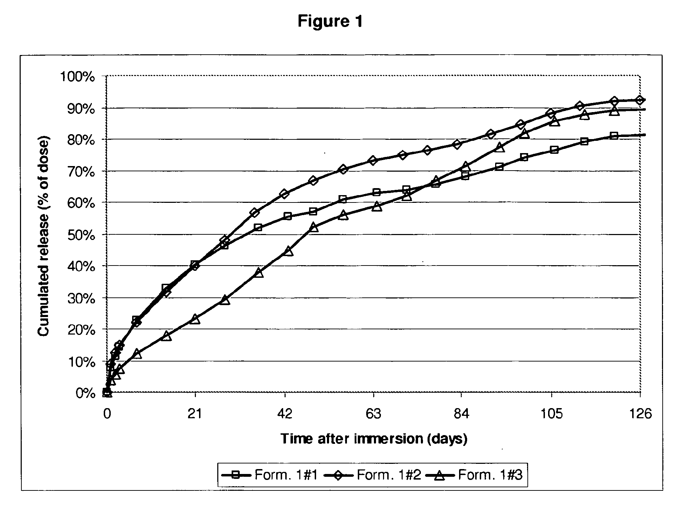

Figure 1 shows, in ordinates, the active ingredient overall release profile expressed in mg versus, in abscissa, the time expressed in days after immersion in the aqueous medium of the subcutaneous implants in the aqueous medium prepared as described in Example 1.

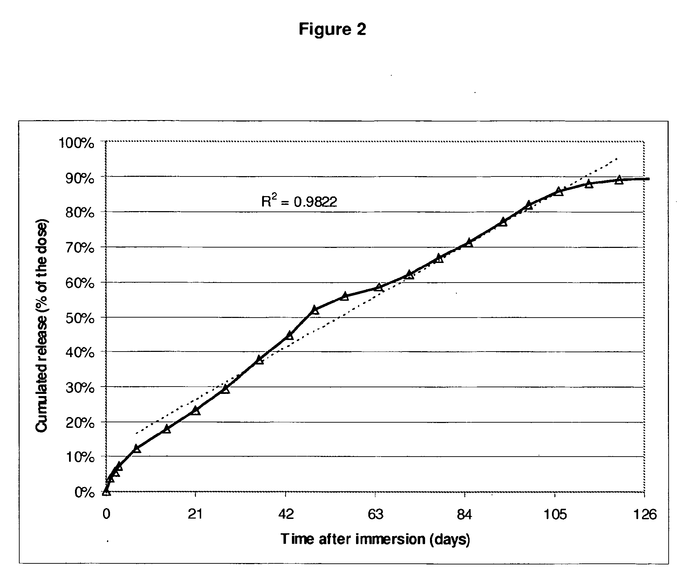

Figure 2 shows, in ordinates, the active ingredient overall release profile expressed in % of total, versus, in abscissa, the time expressed in days after immersion in the aqueous medium of the sole subcutaneous implants 1#3 prepared as described in Example 1.

It is observed from figure 1 that slower release profiles are obtained in the first 3-4 weeks with the subcutaneous implants 1#2 and 1#3 containing the blended extruded PLGA having a resulting weight average molecular weight of 60000 Da and an UG ratio of 59/41 if compared to that obtained with subcutaneous implants MedRH108 containing a single PLGA having the same and aforementioned average molecular weight and UG molar ratio values of the resulting blended PLGA utilised in 1#2 and 1#3. In this case, the formulation (1#3) actually leads to a more fairly linear release profile (R² = 0.9820 for % active ingredient released = f(t) from week 1 to 17 calculated by linear regression - see Figure 2).

### EXAMPLE 2- preparation of subcutaneous implants containing Leuprorelin (formulations No. 2#1 and 2#2)

Subcutaneous implants containing 23.5% w/w Leuprorelin (having particle size distribution ranging from 1 to 100 µm) and PLGA having compositions, UG molar ratios and molecular weights as defined in the table below are prepared as described in WO00/33809

| | PLGA mix composition | Resulting "blended" PLGA | |
|---|---|---|---|
| | | L/G molar ratio | Molecular weight |
| 2#1 | 1 single PLGA | 75/25 UG | 110 kg/mol |
| 2#2 | 2 PLGAs: - 75% m/m of a 75/25 L/G - 110 Kg/mol PLGA - 25% m/m of a 51/49 UG - 18 Kg/mol PLGA | 70/30 L/G | ∼90 kg/mol |

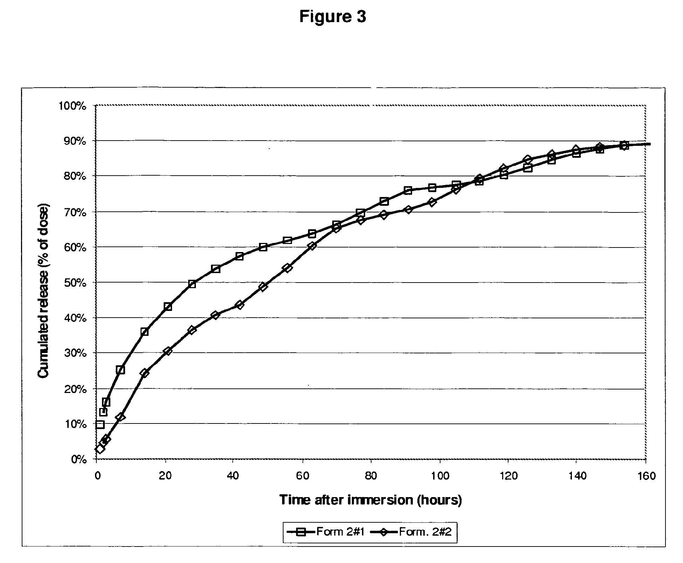

Figure 3 shows, in ordinates, the active ingredient overall release profile expressed in mg, versus, in abscissa, the time expressed in days after immersion in the aqueous medium of the subcutaneous implants prepared as described in Example 2, and in the smaller diagram the active ingredient release profile expressed in mg in the first seven days of release.

It is observed that the addition of a higher Glycolide ratio and lower molecular weight PLGA actually leads to lower initial release rate and to a more linear release profile when compared to the one obtained with a single 75/25 - high molecular weight PLGA.

### EXAMPLE 3- preparation of subcutaneous implants containing Leuprorelin (formulations No. 3#1 and 3#2)

Subcutaneous implants containing 27% w/w Leuprorelin (having particle size distribution ranging from 1 to 100 µm) and PLGA having compositions, UG molar ratios and molecular weights as defined in the table below are prepared as described in WO00/33809

| | PLGA mix composition | Resulting "blended" PLGA | |
|---|---|---|---|
| | | L/G molar ratio | Molecular weight |
| 3#1 | 2 PLGAs: - 75% m/m of a 75/25 L/G - 110 Kg/mol PLGA - 25% m/m of a 51/49 UG - 18 Kg/mol PLGA | 70/30 L/G | 87 kg/mol |
| 3#2 | 2 PLGAs: - 75% m/m of a 75/25 UG - 110 Kg/mol PLGA - 25% m/m of a 100/0 UG - 15 Kg/mol PLGA | 80/20 L/G | ∼90 kg/mol |

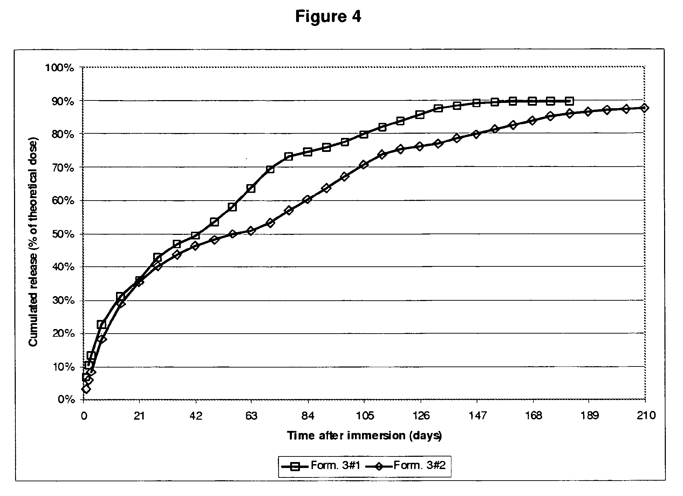

Figure 4 shows, in ordinates, the active ingredient overall release profile expressed in mg versus, in abscissa, the time expressed in days after immersion in the aqueous medium of the subcutaneous implants prepared as described in Example 3. It is observed that the addition of a lower molecular weight PLGA actually leads to lower initial release rate and to longer release duration when compared to the one obtained when adding PLGA with low weight average molecular weight and 51/49 UG PLGA to the PLGA having a UG of 75/25 and high molecular weight.

### EXAMPLE 4- preparation of subcutaneous implants containing Fentanyl citrate

Subcutaneous implants containing 40.0% m/m Fentanyl citrate (having particle size distribution ranging from 1 to 63 µm) and PLGA having compositions, UG molar ratios and molecular weights as defined in the table below are prepared as described in WO00/33809

| Formulation Nr | PLGA mix composition | Resulting "blended" PLGA | |
|---|---|---|---|
| | | L/G molar ratio | Molecular weight |
| 4#1 | 1 single PLGA | 54/46 | 51 kg/mol |
| 4#2 | 2 PLGAs: - 75% m/m of a 54/46 L/G - 51 Kg/mol PLGA - 25% m/m of a 50/50 UG - 17 Kg/mol PLGA | 53/47 ≈ | 42 kg/mol |

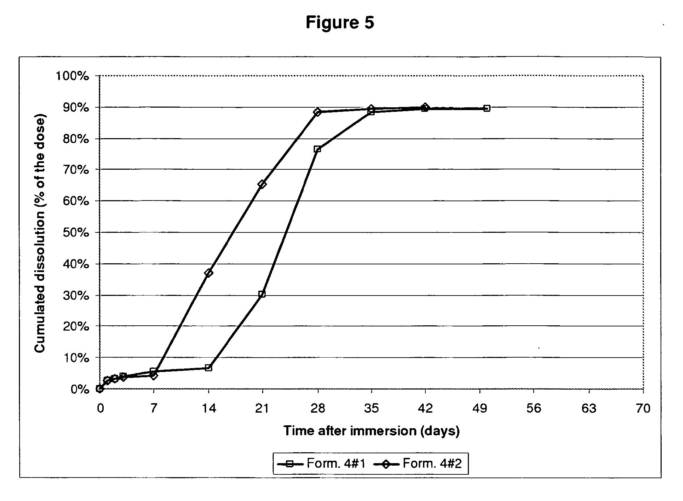

Figure 5 shows the active ingredient release profiles (% of the dose released versus time after immersion) of the various implants of Example 4.

This example demonstrates that, if compared to the release profile single PLGA (Form. 4#1), the formulation (Form. 4#2) allows to achieve a more linear release profile also between the seventh and 25^{th} day.

### EXAMPLE 5- preparation of subcutaneous implants containing Medroxy Progesterone Acetate

Subcutaneous implants containing 55% m/m Medroxy Progesterone Acetate (having particle size distribution ranging from 1 to 63 µm) and PLGA having compositions, UG molar ratios and molecular weights as defined in the table below are prepared as described in WO00/33809

| Formulation Nr | PLGA mix composition | Resulting "blended" PLGA | |
|---|---|---|---|
| | | L/G molar ratio | Molecular weight |
| 5#1 | 1 single PLGA | 75/25 | 120 kg/mol |
| 5#2 | PLGAs: - 50% m/m of a 75/25 UG - 120 Kg/mol PLGA - 50% m/m of a 60/40 UG - 53 Kg/mol PLGA | 67.5/32.5 | ≈ 85 kg/mol |

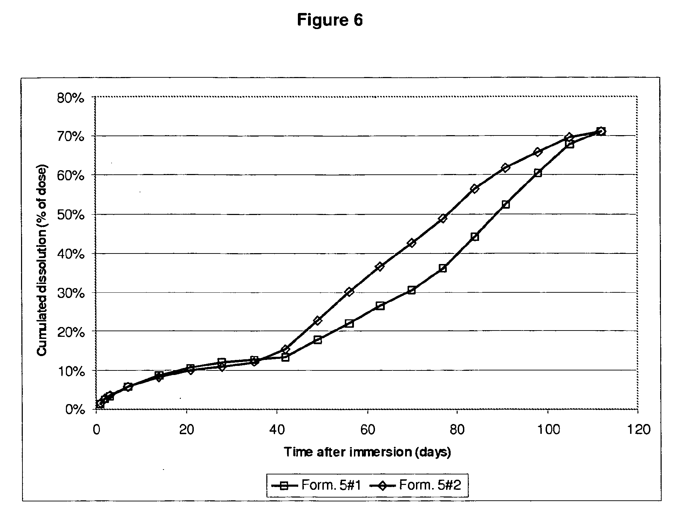

Figure 6 shows the active ingredient release profiles (% of the dose released versus time after immersion) of the various implants from example 5.

As it results from a comparison of the two release profiles it results that the formulation 5# 2 also after the 40th day of the invention a more linear release profile can be achieved with the subcutaneous implants containing a mixture of PLGA than with the subcutaneous implants containing a single PLGA, having a similar UG molar ratio and an approximately similar average molecular weight.

## Claims

1. Subcutaneous implants obtained by extrusion containing an active ingredient dispersed in a PLGA matrix, wherein said matrix is obtained by grinding an extruded product consisting of a blend of:
□ at least two PLGA having different lactic acid/glycolic acid molar ratios and different weight average molecular weights, or
□ a PLGA and PLA having different weight average molecular weight,
wherein said active ingredient selected from the group consisting of: a peptide, an active ingredient able to increase bone density chosen from: pharmaceutically acceptable bisphosphonic acids and their salts, vitamin D and sex hormones, an analgesic-narcotic active principle, a steroid hormone for hormone treatments during menopause and for contraception.

2. Subcutaneous implants according to claim 1 wherein the peptide is selected from the group consisting of avorelin, triptorelin, goserelin and leuprorelin.

3. Subcutaneous implants according to claim 1 wherein these biphosponic acid salts are selected from disodium etidronate, disodium alendronate and disodium pamidronate.

4. Subcutaneous implants according to claim 1 , wherein said sex hormones are selected from the group consisting of: estrogens and androgenic progestins.

5. Subcutaneous implants according to claim 4, wherein said estrogens are chosen from the class consisting of estradiol, estradiol valerate, estradiol cypionate, estrone, estrone sulphate or estrogens of non-steroidal type.

6. Subcutaneous implants according to claim 4 wherein said androgenic progestins are chosen from the class consisting of norethindrone, norethinodrel, norgestrel, desogestrel, norgestimate.

7. Subcutaneous implants according to claim 1, wherein the active ingredient with narcotic analgesic activity is selected from the group consisting of morphine and morphinans, and µ receptor agonists.

8. Subcutaneous implants according to claim 7, wherein said µ receptor agonists are phenylpiperidines selected from the group consisting of: meperidine, fentanyl and relative pharmaceutically acceptable salts, fentanyl congeners selected from sufentanyl, alfentanyl, lofentanyl, carfentanyl, remifentanyl and their pharmaceutically acceptable salts.

9. Subcutaneous implants according to anyone of claims 1-8 wherein the active ingredient therein contained has homogeneous or heterogeneous particles size distribution.

10. Subcutaneous implants according to claim 9, wherein, when the active ingredient is a peptide, it shows a heterogeneous particles size distribution comprised between 1 and 63 µm or between 1 and 100 µm.

11. Subcutaneous implants according to claim 10, wherein the resulting extruded blended PLGA has a lactic acid / glycolic acid molar ratio ranging from 50/50 and 90:10 and the weight average molecular weight ranges from 50000 to 150000.

12. A process for preparing the subcutaneous implants according to anyone of claims 1-11 comprising the following steps
a) mixing at least two PLGA having different weight average molecular weight and different lactic acid/ glycolic acid molar ratios,
b) extruding the powder mix coming from step (a) and then grinding the extruded PLGA mixture, thereby obtaining the blended extruded PLGA granules
c) dry mixing the active agent in the form of particles with the granules of said blended extruded PLGA coming from step (b), or (c') wet granulating the active ingredient particles and the granules of the blended extruded PLGA coming from step (b), using a suitable solvent,
d) drying the granulated product coming from wet granulation of step (c) thereby obtaining a residue containing a maximum liquid content of between 0.1 and 3%,
e) extruding the dry mixture coming from step (c) or the dried granulated product from step (d).

## Patentansprüche

1. Subkutane Implantate, die durch Extrusion erhalten werden und einen aktiven Wirkstoff enthalten, der in einer PLGA-Matrix dispergiert ist, worin die Matrix erhalten wird durch Mahlen eines extrudierten Produkts, das besteht aus einer Mischung von:
- wenigstens zwei PLGA, die unterschiedliche Mol-Verhältnisse Milchsäure/Glycolsäure und unterschiedliche Gewichtsmittel des Molekulargewichts aufweisen; oder
- einem PLGA und einem PLA, die unterschiedliche Gewichtsmittel des Molekulargewichts aufweisen,
worin die aktive Komponente gewählt ist aus der Gruppe, die besteht aus einem Peptid, einer aktiven Komponente, die in der Lage ist, die KnochenDichte zu erhöhen, gewählt aus pharmazeutisch annehmbaren Biphosphonsäuren und ihren Salzen, Vitamin D und Sexualhormonen, einem analgetisch-narkotisch aktivem Prinzip, einem Steroid-Hormon für Hormon-Behandlungen während der Meno-Pause und für eine Empfängnisverhütung.

2. Subkutane Implantate gemäß Anspruch 1, worin das Peptid gewählt ist aus der Gruppe, die besteht aus Avorelin, Triptorelin, Goserelin und Leuprorelin.

3. Subkutane Implantate gemäß Anspruch 1, worin diese Biphosphonsäure-Salze gewählt sind aus Dinatrium-Etidronat, Dinatrium-Alendronat und Dinatrium-Pamidronat.

4. Subkutane Implantate gemäß Anspruch 1, worin die Sexualhormone gewählt sind aus der Gruppe, die besteht aus Östrogenen und androgenen Progestinen.

5. Subkutane Implantate gemäß Anspruch 4, worin die Östrogene gewählt sind aus der Klasse, die besteht aus Östradiol, Östradiol-Valerat, Östradiol-Cypionat, Östron, Östron-Sulfat oder Östrogenen des nicht-steroidalen Typs.

6. Subkutane Implantate gemäß Anspruch 4, worin die androgenen Progestinen gewählt sind aus der Klasse, die besteht aus Norethindron, Norethinodrel, Norgestrel, Desogestrel und Norgestimat.

7. Subkutane Implantate gemäß Anspruch 1, worin der aktive Wirkstoff mit narkotisch-analgetischer Aktivität gewählt ist aus der Gruppe, die besteht aus Morphin und Morphinanen und µ-Rezeptor-Agonisten.

8. Subkutane Implantate gemäß Anspruch 7, worin die µ-Rezeptor-Agonisten Phenylpiperidine sind, die gewählt sind aus der Gruppe, die besteht aus Meperidin, Fentanyl und verwandten pharmazeutisch annehmbaren Salzen, Fentanyl-Artverwandten, die gewählt sind aus Sufentanyl, Alfentanyl, Lofentanyl, Carfentanyl, Remifentanyl und ihren pharmazeutisch annehmbaren Salzen.

9. Subkutane Implantate gemäß irgendeinem der Ansprüche 1 bis 8, worin die aktive Komponente, die darin enthalten ist, eine homogene oder heterogene Teilchengröße-Verteilung aufweist.

10. Subkutane Implantate gemäß Anspruch 9, worin dann, wenn die aktive Komponente ein Peptid ist, diese eine heterogene Teilchengröße-Verteilung zeigt, die zwischen 1 und 63 µm oder zwischen 1 und 100 µm liegt.

11. Subkutane Implantate gemäß Anspruch 10, worin die resultierende extrudierte gemischte PLGA ein Mol-Verhältnis Milchsäure/Glycolsäure aufweist, das im Bereich von 50/50 und 90:10 liegt, und das Gewichtsmittel des Molekulargewichts im Bereich von 50.000 bis 150.000 liegt.

12. Verfahren zur Herstellung der subkutanen Implantate gemäß irgendeinem der Ansprüche 1 bis 11, umfassend die folgenden Schritte:
a) Mischen von wenigstens zwei PLGA, die unterschiedliche Gewichtsmittel des Molekulargewichts und unterschiedlichem Mol-Verhältnis Milchsäure/Glycolsäure aufweisen;
b) Extrudieren der Pulvermischung, die aus Schritt a) stammt, und anschließendes Mahlen der extrudierten PLGA-Mischung, wodurch die gemischten extrudierten PLGA-Granulate erhalten werden;
c) Trockenmischen des aktiven Mittels in Form von Teilchen mit den Granulaten des gemischten extrudierten PLGA, das aus Schritt b) stammt; oder c') Nassgranulieren der Teilchen der aktiven Komponente und der Granulate des gemischten extrudierten PLGA, die aus Schritt b) stammen, unter Verwendung eines geeigneten Lösungsmittels;
d) Trocknen des granulierten Produkts, das aus der Nass-Granulation von Schritt c) stammt, wodurch man einen Rückstand erhält, der einen maximalen Flüssigkeitsgehalt von zwischen 0,1 und 3 % aufweist;
e) Extrudieren der aus Schritt c) stammenden trockenen Mischung oder des getrockneten granulierten Produkts aus Schritt d).

## Revendications

1. Implants sous-cutanés obtenus par extrusion, contenant un ingrédient actif dispersé dans une matrice de PLGA, dans lesquels ladite matrice est obtenue en broyant un produit extrudé constitué d'un mélange de :
- au moins deux PLGA ayant différents rapports molaires acide lactique/acide glycolique et différents poids moléculaires moyens en poids, ou
- un PLGA et un PLA ayant différents poids moléculaires moyens en poids,
dans lesquels ledit ingrédient actif choisi parmi le groupe constitué de : un peptide, un ingrédient actif pouvant augmenter la densité osseuse choisi parmi : des acides bisphosphoniques pharmaceutiquement acceptables et leurs sels, la vitamine D et des hormones sexuelles, un principe actif analgésique-narcotique, une hormone stéroïde pour les traitements hormonaux pendant la ménopause et pour la contraception.

2. Implants sous-cutanés selon la revendication 1, dans lesquels le peptide est choisi parmi le groupe constitué d'avoréline, de triptoréline, de goséréline et de leuproréline.

3. Implants sous-cutanés selon la revendication 1, dans lesquels ces sels d'acides bisphosphoniques sont choisis parmi l'étidronate disodique, l'alendronate disodique et le pamidronate disodique.

4. Implants sous-cutanés selon la revendication 1, dans lesquels lesdites hormones sexuelles sont choisies parmi le groupe constitué d'estrogènes et de progestines androgéniques.

5. Implants sous-cutanés selon la revendication 4, dans lesquels lesdits estrogènes sont choisis parmi la classe constituée d'estradiol, de valérate d'estradiol, de cypionate d'estradiol, d'estrone, de sulfate d'estrone ou d'estrogènes de type non stéroïdien.

6. Implants sous-cutanés selon la revendication 4, dans lesquels lesdites progestines androgéniques sont choisies parmi la classe constituée de noréthindrone, de noréthinodrel, de norgestrel, de désogestrel ou de norgestimate.

7. Implants sous-cutanés selon la revendication 1, dans lesquels l'ingrédient actif ayant une activité narcotique-analgésique est choisi parmi le groupe constitué de morphine et de morphinanes, et d'agonistes aux récepteurs µ.

8. Implants sous-cutanés selon la revendication 7, dans lesquels lesdits agonistes aux récepteurs µ sont des phénylpipéridines choisies parmi le groupe constitué de : mépéridine, fentanyl et sels relatifs pharmaceutiquement acceptables, congénères du fentanyl choisis parmi le sufentanyl, l'alfentanyl, le lofentanyl, le carfentanyl, le rémifentanyl et leurs sels pharmaceutiquement acceptables.

9. Implants sous-cutanés selon l'une quelconque des revendications 1 à 8, dans lesquels l'ingrédient actif contenu à l'intérieur de ceux-ci a une répartition en taille de particules homogène ou hétérogène.

10. Implants sous-cutanés selon la revendication 9, dans lesquels, lorsque l'ingrédient actif est un peptide, il présente une répartition en taille de particules hétérogène comprise entre 1 et 63 µm ou entre 1 et 100 µm.

11. Implants sous-cutanés selon la revendication 10, dans lesquels le PLGA mélangé extrudé résultant a un rapport molaire acide lactique/acide glycolique variant de 50/50 à 90:10 et le poids moléculaire moyen en poids varie de 50 000 à 150 000.

12. Procédé pour préparer les implants sous-cutanés selon l'une quelconque des revendications 1 à 11, comportant les étapes suivantes consistant à :
a) mélanger au moins deux PLGA ayant différents poids moléculaires moyens en poids et différents rapports molaires acide lactique/acide glycolique,
b) extruder le mélange en poudre issu de l'étape (a), puis broyer le mélange de PLGA extrudé, en obtenant ainsi les granules de PLGA extrudés mélangés,
c) mélanger à sec l'agent actif sous la forme de particules avec les granules dudit PLGA extrudé mélangé issus de l'étape (b), ou (c') granuler par voie humide les particules d'ingrédient actif et les granules du PLGA extrudé mélangé issus de l'étape (b), en utilisant un solvant adapté,
d) sécher le produit granulé issu de la granulation par voie humide de l'étape (c) en obtenant ainsi un résidu contenant une teneur en liquide maximale comprise entre 0,1 et 3 %,
e) extruder le mélange sec issu de l'étape (c) ou le produit granulé séché de l'étape (d).
